# EUROPEAN PATENT APPLICATION

(11) **EP 1 340 503 A1**
(43) Date of publication of application: **03.09.2003**
(21) Application number: 02004787.4
(22) Date of filing: 01.03.2002
(51) Int. Cl.: A61K 31/565, A61K 31/566, A61K 31/567, A61P 5/30, A61K 9/12

(54) **Solution aerosol formulation containing esters of 3, 17-dihydroxy oestratriene derivates for pulmonary delivery**

(71) Applicant: CHIESI FARMACEUTICI S.p.A., 43100 Parma (IT)
(72) Inventor: Davies, Rebecca Jaine, 43100 Parma (IT); Ganderton, David, 43100 Parma (IT); Lewis, David Andrew, 43100 Parma (IT); Meakin, Brian John, 43100 Parma (IT); Brambilla, Gaetano, 43100 Parma (IT); Ferraris, Alessandra, 43100 Parma (IT)
(74) Representative: Minoja, Fabrizio, Dr.

(57) **Abstract**

The invention discloses formulations for administration with pressurized metered dose inhalers containing as active ingredient esters of 3,17-dihydroxy oestratriene derivatives, in solution in a hydrofluorocarbon propellant, optionally one or more co-solvents, and their use for the treatment of postmenopausal symptoms as well as of menstrual migraine headaches.

## Description

### BACKGROUND OF THE INVENTION

Hormone replacement therapy (HRT) is the term used to describe the therapeutic use of oestrogen in postmenopausal women. HRT is indicated for women whose lives are inconvenienced by vaginal atrophy or vasomotor instability. Natural oestrogens such as 17β-oestradiol (also indicated hereinafter as oestradiol *tout court*) and oestriol as well as synthetic analogues such as ethinyl oestradiol and mestranol, whose formulae are reported below, are used to treat menopausal symptoms. Females are classed as postmenopausal if serum oestradiol levels are less than 20pg/ml (Baracat *et al., Curr Therap Res* 1999, 60, 129-137). 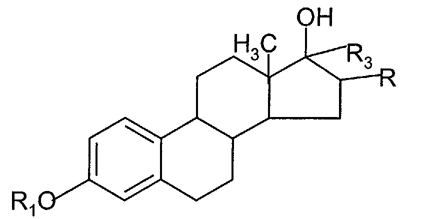
17β-Oestradiol: R = R₁ = R₃ = H
Oestriol: R= OH; R₁ = R₃ = H
Ethinyl oestradiol: R= R₁ = H; R₃ = ethynyl
Mestranol:R =H; R₁ = methyl; R₃ = ethynyl

HRT is also a common means of preventing and treating established osteoporosis. Women who receive oestrogen early in the menopause and continue with it for six to nine years, can reduce their overall risk of fracture by 50 per cent (Lindsay, *Osteporosis Int* 1993, 3, S9-12). A 0.625 mg dosage of oral conjugated oestrogen per day (or its equivalent) is the minimum dose required to protect against osteoporotic fractures. Lower doses of oestrogen (0.3 mg per day) combined with calcium supplements (1,500 mg per day) may also protect bones.

Another possible role for oestrogens is in the treatment of menstrual migraine headaches. Between four to seven percent of migraine sufferers have a diagnosis of menstrual migraine. A fall in oestrogen levels has been associated with the development of migraine (MacGregor *et al., Cephalalgia* 1990, 10, 305-310). Others have also reported that the premenstrual administration of oestrogens prevented the headache without affecting the menses (DeLignieres et al., *Brit Med J* 1986, 293, 1540).

The most widely utilised oestrogens are 17β-oestradiol and its derivatives.

Various kinds of 17β-oestradiol formulations have been reported so far such as oral, transdermal and reservoir patches, topical sprays, implants, sublingual, lingual sprays, injectables and intranasal formulations, either as powders and aqueous formulations.

A short survey is reported as follows.

Oral preparations of oestradiol derivatives contain 1mg or 2mg of either 17β-oestradiol or β-oestradiol-17-valerate (with or without a progesterone). Transdermal patches contain oestradiol and can release approximately 25 to 100µg over a 24 hours period. Reservoir patches are available which are applied every 3-4 days or matrix patches which are worn consecutively for seven days. Typical mean serum oestradiol levels obtained with these patches are 70-90pg/ml (Chehkowski et al. *NEMJ,* 1986, 324, 1615-1620). Patches enable stable serum concentrations to be achieved; however they can frequently irritate the skin. The skin is also impermeable to progesterone (Flynn, in Guy, R. ed. *Percutaneous Absorption* 1989, pp. 27-51), making co-delivery with oestradiol difficult to achieve. A 25, 50 and 100mg Oestradiol implant is also available which is replaced every 4-8 months. The recurrence of vasomotor symptoms at supraphysiological plasma concentrations and prolonged endometrial stimulation after discontinuation of the implants have been encountered.

In WO 00/45795 topical spray compositions, in the form either solution or suspension comprising one or more medicaments, including oestradiol, in a volatile vehicle, and one or more film-forming polymers are disclosed. The vehicle can be water or a non-aqueous solvent. If desired, such compositions may also comprise a propellant. When sprayed on a topical site, the composition forms a stable, breathable film from which the medicaments are transdermally available. Among the examples, a formulation comprising oestradiol, PVP K-30, vinylacetate-vinylpyrrolidone copolymer, polyethylene glycol and dichlorodifluoromethane/trichloromonofluoromethane as propellants is disclosed.

As far as injectable formulations are concerned, several esters of 17β-oestradiol have been employed . Martindale: The Extra Pharmacopoeia, 26th Ed. (1972) contains formula for oestradiol benzoate, cypionate, dipropionate, enanthate and undecanoate injections. The usual dosage is 1 to 5mg intramuscularly every 1 to 2 weeks. β-Oestradiol 3,17-dipropionate Injection (B.P. 1948) is a sterile solution in ethyl oleate or a suitable oil, +containing 1mg β-oestradiol 3,17-dipropionate in 1 ml (0.1% w/v).

Others have investigated the pharmacokinetic profile of micronised 17β-oestradiol administered by the sublingual route (Price *et al.. Obstetrics & Gynecology* 1997, 89, 340-345). With sublingual administration of 0.25, 0.5 and 1.0mg of 17β-oestradiol, peak plasma concentrations of 294, 245 and 451 pg/ml were achieved respectively within 1 hour. The 17β-oestradiol levels returned to less than 60pg/ml after 6 hours.

Lingual spray formulations of 17β-oestradiol have been proposed by Flemington for the treatment of postmenopausal vasomotor symptoms exploiting a proprietary technology based on the use of non-polar solvents such as Miglyol. A rapid and efficient absorption of oestradiol into the blood will be of benefit for rapidly relieving hot flushes.

EP 242643 refers to compositions for intranasal administration comprising an aqueous solution of a peptide or a steroid, including 17β-oestradiol, polysorbate 80 and a nonionic surfactant which is able to enhance the absorption of the peptide or of the steroid. According to the inventors, said formulations cause a reduced irritation of the nasal mucosa.

Intranasal administration of aqueous solutions of 17β-oestradiol with dimethyl-β-cyclodextrin as an absorption enhancer has also been investigated (Schipper *et al., Int J Pharm* 1990, 64, 61-66). This study resulted in a bioavailability of approximately 65%, compared to the IV route. The nasal preparation contained 500µg/ml of 17β-oestradiol in a 20µl dose.

Some of the problems encountered with the current modes of delivering oestrogens could be overcome by utilizing the pulmonary route. The main advantages are the absence of first-pass hepatic metabolism and the large absorption surface provided by the respiratory airways (Altiere and Thompson, In Hickey, A. J., ed. *Inhalation aerosols: Physical and biological basis for therapy,* 1996, pp. 85-137). For instance, powder formulations, obtained by spray drying oestradiol with various excipients (large porous particles) have been successfully administered to rats by inhalation (Wang *et al., J Aerosol Med* 1999, 12, 27-36). In their study, 6mg of said porous particles containing 600µg of 17β-oestradiol showed sustained delivery for up to 5 days. The relative bioavailability of the inhalation aerosol when compared to subcutaneous injection was 86%. The fine particle fraction obtained for the 17β-oestradiol porous particles using Andersen cascade Impaction was approximately 33%. Preliminary human studies performed with the 17β-oestradiol porous particles indicate that inhaled doses in the 0.5 to 5mg range can lead to elevated oestradiol level in humans, with pharmacokinetics similar to those of the 3-4 day transdermal patch.

It would be even more desirable to provide formulations for the pulmonary delivery of oestrogens as a solution since it would allow a more rapid and efficient delivery to the systemic circulation . The rapid absorption of oestrogens into the blood would advantageously allow reduction of pain associated with menstrual migraine headaches. It could also remove the need for continuous dosing of oestrogens in the treatment of vasomotor symptoms in postmenopausal women allowing dosing *'when required'.* In addition it could be used as an 'add-on therapy' in those whose symptoms are not fully alleviated by continuous therapy. In comparison to powder formulations, solution formulations could also guarantee a higher dose reproducibility after repeated administrations as well as the possibility of achieving a finer control of the size of the particles.

Pharmaceutical products in solution could be administered to the respiratory tract by using pressurised metered dose inhalers (pMDIs).PMDIs use a propellant to expel droplets containing the pharmaceutical product to the respiratory tract as an aerosol.

As far as the type of propellant is concerned, hydrofluoroalkanes [(HFAs) known also as hydro-fluoro-carbons (HFCs)] would be mandatory propellants as chlorofluorocarbons (known also as Freons or CFCs), which were for many years the preferred propellants for pharmaceutical use, have been implicated in the destruction of the ozone layer so their use is being phased out.

In particular, 1,1,1,2-tetrafluoroethane (HFA 134a) and 1,1,1,2,3,3,3-heptafluoropropane (HFA 227) have been acknowledged to be the best candidates for non-CFC propellants and a number of medicinal aerosol formulations using such HFA propellant systems have been disclosed.

Documents of the prior art dealing with preparations for inhalation of 17β-oestradiol through pMDIs are the following:

US 4895719 in which 17β-oestradiol is proposed as active ingredient, discloses dehydrated liposome particles in suspension in CFC propellants.

GB 1099722 discloses aerosol solution preparations, which comprise (a) a steroid and/or an antibiotic which is insoluble or sparingly soluble in water and oil; (b) a copolymer of vinyl pyrrolidone and vinyl acetate (PVP/VA); (c) methanol, ethanol, isopropanol, ethylene glycol monomethyl ether or ethylene glycol monoethyl ether; and (d) a CFC or a hydrocarbon propellant. Oestradiol and ethynyl-oestradiol are mentioned among the possible active ingredients.

WO 98/34595 refers to aerosol formulations in the form of solutions or suspensions in which the propellant is a mixture of a HFA and carbon dioxide. Example 9 discloses a formulation containing 0.037% w/w oestradiol, 0.0036% w/w oleic acid, 5.7% w/w ethanol in HFA 134a and carbon dioxide. Percentages are given as the total weight of the formulation. It is not specified if the formulation is in the form of a solution or suspension.

However, said concentration is unlikely to give the serum oestradiol levels necessary to produce a therapeutic effect.

### Disclosure of the invention

The aim of providing solution formulations of 17β-oestradiol in an HFA propellant for aerosol delivery is to give a rapid systemically active dose of said drug *via* the respiratory tract.

In order to provide an efficient aerosol delivery of the drugs, therapeutically useful concentrations of 17β-oestradiol should be achieved.

Another very important parameter is the size of the aerosol liquid droplets provided by the pMDI, normally expressed as mass median aerodynamic diameter (MMAD). Said size should be small enough to be delivered to the lungs and to be absorbed into the bloodstream upon inhalation, i.e. advantageously comprised between about 0.5 µm and 2.5 µm (MMAD of about 1-2 µm). Particles of size smaller than 0.5 µm are indeed not therapeutically useful as they are exhaled.

The aerosol formulations in solution offer the advantage of being homogeneous, with the active ingredient and excipients completely dissolved in the propellant vehicle or its mixture with suitable co-solvents such as ethanol. Solution formulations also obviate physical stability problems associated with suspension formulations so assuring reproducible delivering of the dose.

Furthermore, when a systemic effect is required, as in the case of the invention, aerosol solution formulations offer the advantage that much finer clouds, largely defined by the drug concentration in the solution, are generated and the finer clouds give more extensive deposition in the lung periphery.

17 β-oestradiol is slightly soluble in HFA propellants such as HFA 134a and HFA 227 or in their mixture with small amounts of co-solvents such as ethanol.

In order to make homogeneous solution formulations containing potentially therapeutic concentrations of oestradiol (higher than 0.05% w/v), large amounts of co-solvents, usually ethanol, are required. A large amount of ethanol, in turn, increases, proportionally to its concentration, the size of the aerosol droplets leaving the actuator orifice. The larger size droplets extensively deposit into the oropharyngeal tract to the detriment of the dose fraction of drug which penetrates into the lower airways (respirable fraction). A poor respirable fraction is unlikely to give the serum oestradiol levels necessary to produce a therapeutic effect.

Moreover, as the amount of ethanol in the formulation increases so does the amount of residual water. High water content, in turn either could lead to unacceptable levels of chemical degradation and could be detrimental to the physical stability of the formulation giving rise to a non-homogeneous system.

In view of the intrinsic poor solubility of 17β-oestradiol, in pMDI vehicles it is necessary to resort to derivatives in which the polarity of the parent molecule is reduced. Besides being less polar, said derivatives should also promptly release the active moiety (parent drug) allowing it to act rapidly and efficiently when delivered to the lung.

However 3-acyl derivatives commercially available, such as 3-benzoate, are not suitable as they suffer from the same solubility problems as the parent drug. Therefore they cannot be formulated as solutions without the use of large amounts of co-solvents.

In consideration of all the problems and disadvantages connected with the available formulations of oestrogens, it would be advantageous to provide a formulation for pulmonary delivery to be used with pressurised metered dose inhalers, which is chemically and physically stable and capable of providing, on actuation, a respirable fraction which in turn, could rapidly give rise to therapeutical plasma levels of the oestrogen. The delivered respirable fraction should be at least 50%, preferably more than 60%, even more preferably more than 70% of the delivered dose.

It would also be highly advantageous to provide formulations whose delivered dose is very reproducible after repeated administrations from the pMDI.

Since a high systemic exposure of the aerosol particles would, in this case, be of benefit, it would be even more advantageous to provide a formulation wherein the composition of the whole solvent system has been adjusted in order to allow the generation of aerosol particles which could give rise, in turn, to a deep lung penetration, at the same time minimizing the amount of very small particles (≤ 0.5 µm) which would be exhaled.

In order to fulfill the therapeutic requirements outlined above as well as overcoming the technical problems in making oestrogen-based solution formulations for pulmonary delivery, it is necessary to provide solution formulations comprising pro-drugs of 3,17 dihydroxy oestratriene derivatives (I). In particular, there is a need to provide a solution formulation fulfilling the aforementioned requirements and comprising a pro-drug of the oestratriene derivative, an aerosol propellant and a suitable amount of a co-solvent.

Said formulations would be useful for the treatment of postmenopausal symptoms as well as of menstrual migraine headaches.

### DESCRIPTION OF THE INVENTION

The invention refers to aeresol solution pharmaceutical formulations, comprising an ester of 3,17-dihydroxy oestratriene derivatives as active ingredient in a concentration of at least 0.01% w/v in solution in a mixture comprising an hydrofluoroalkane propellant and one or more co-solvents.

More particularly, the invention refers to solution formulations comprising at least 0.01% w/v of an ester of 3,17-dihydroxy oestratriene derivatives of formula (I), an HFA propellant and optionally an amount of one or more co-solvents less than or equal to 25% w/w. 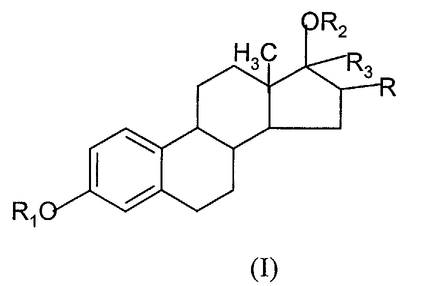 where
R is H or OH;
R₁ is H, C₁-C₄ alkyl, C₁-C₁₀ alkanoyl or benzoyl;
R₂ is C₁-C₁₀ alkanoyl or (C₅-C₆)cycloalkyl(C₁-C₅)alkanoyl;
R₃ is H or ethynyl.

When R₁ is H, the most representative oestratriene esters are those in which R₂ has five or more carbon atoms.

Said solutions are chemically stable for an adequate time and capable of providing, on actuation, a respirable fraction which, in turn, could rapidly give rise to therapeutical plasma levels of the parent drug.

It has indeed been found in accordance with the present invention that, due to the higher solubility of the derivatives of formula (I) in the HFA propellants, higher concentrations of the drug can be obtained, making pulmonary administration of esters of 3,17-dihydroxy oestratriene derivatives particularly advantageous.

According to a preferred embodiment of the invention there is provided a pharmaceutical solution composition comprising at least 0.05% w/v of 3,17-dihydroxy-oestartriene derivatives, particularly β-oestradiol-17 esters of the formula (I) where R, R₁ and R₃ are hydrogen and R₂ is C₄H₉CO-(valeryl) or C₆H₁₃CO- (enanthyl), an HFA propellant and an amount of ethanol less than 10% w/w and an amount of another co-solvent with a higher polarity than ethanol comprised between 0.1% and 2% w/w, preferably between 0.2% and 1% w/w. The addition of a co-solvent with a higher polarity than ethanol, such as propylene glycol or glycerol allows reduction in the amount of ethanol and hence the amount of residual water which could adversely affect the homogeneity of the solution.

According to an even more preferred embodiment of the invention, there is also provided a pharmaceutical composition consisting essentially of either β-oestradiol-3,17-dipropionate or the 17-valerate, an HFA propellant, and optionally an amount of ethanol comprised between 2 and 8% w/w, preferably equal to 5% w/w. In this case, the amount of the active ingredient would be preferably comprised between 0.1 and 1.0% w/v.

It has been indeed found that, although a co-solvent is not needed to dissolve β-oestradiol-3,17-dipropionate in the propellant in the range of concentration indicated, a small amount of ethanol (preferably around 5% w/w) produces the deposition characteristics more likely to achieve systemic delivery . Thus, ethanol does not act as a co-solvent, but rather helps to reduce the amount of very small particles (0.5µm) which would be exhaled due to a short residency time in the lung. Moreover, ethanol reduces deposition on the actuator orifice of the inhaler so improving the reproducibility of the dose after repeated administrations by keeping *'clean'* the actuator orifice used for dispersing the formulation to a patient.

According to a further aspect of the invention there is provided a method of filling an aerosol inhaler with a composition of the invention, the method comprising:
(a) weighing the required quantity of active ingredient into the can or vial;
(b) adding the appropriate volume of ethanol and the other co-solvent, if required;
(c) crimping with valves and gassing
(d) adding a propellant containing a hydrofluoroalkane (HFA);

In the prior art, esters of 17β-oestradiol have been only formulated as injectables in oily solution and no document discloses solution formulations containing them for aerosol delivery. In WO 98/56349 the Applicant disclosed solution compositions for use in an aerosol inhaler, comprising an active ingredient, a propellant containing a hydrofluoroalkane (HFA), a cosolvent and further comprising a low volatility component to increase the mass median aerodynamic diameter (MMAD) of the aerosol particles on actuation of the inhaler. Esters of oestradiol are not mentioned even less exemplified. On the other hand the administration of esters of steroids by pulmonary route may present concerns about the possibility of release of the active moiety through de-esterification.

Accordingly, a further aspect of the invention is directed to the use of an ester of 3,17-dihydroxy oestratriene and in particular of 17β-oestradiol for preparing aerosol solution formulation for the treatment of postmenopausal symptoms and menstrual migraine headaches.

### DETAILED DISCLOSURE OF THE INVENTION

Active ingredients which may be used in the aerosol compositions of the invention are pro-drugs of 3,17-dihydroxy oestratriene derivatives, preferably esters of 3,17-dihydroxy oestratriene derivatives of formula I 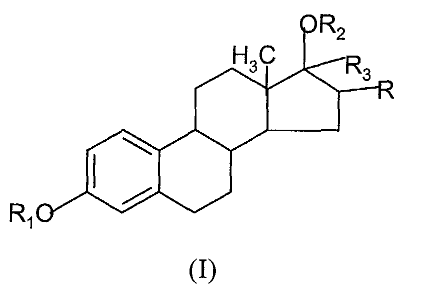 where
R is H or OH
R₁ is H, C₁-C₄ alkyl, C₁-C₁₀ alkanoyl or benzoyl and preferably a C₂-C₇ alkanolyl
R₂ is a C₁-C₁₀ alkanoyl or a (C₅-C₆)cycloalkyl(C₁-C₅)alkanoyl and preferably a C₂-C₇ alkanolyl,
R₃ is H or ethynyl.

The preferred esters of oestratriene derivatives are those in which R₁ and R₂ are C₃ alkanoyl groups or R₁ is H and R₂ has five or more carbon atoms.

Even more preferred are -oestradiol-17-valerate or -oestradiol-3,17-dipropionate (R=R₃=H).

Advantageously, the concentration of the active ingredient is at least 0.01% w/v, preferably at least 0.05% w/v, more preferably at least 0.1% w/v, even more preferably between 0.1 and 1.0% w/v.

It is preferable that the formulation is suitable for delivering a therapeutic amount of the active ingredient in one or two actuations of the pMDI. Advantageously the formulation will be suitable for delivering at least 25 µg/dose, preferably between 50 and 500 µg/dose. By "dose" it is meant the amount of active ingredient delivered by a single actuation of the inhaler.

The formulations of the invention could be filled into cans suitable for delivering pharmaceutical aerosol formulations, preferably into cans having part of all of the internal surfaces made of anodised aluminium, stainless steel or lined with an inert organic coating. Examples of preferred coatings are epoxy-phenol resins, perfluoroalkoxyalkanes, perfluoroalkoxyalkylenes, perfluoroalkylenes such as polytetrafluoroethylene, fluorinated-ethylene-propylene, polyether sulfone and a copolymer, fluorinated-ethylene-propylene polyether sulfone. Other suitable coatings could be polyamide, polyimide, polyamideimide, polyphenylene sulfide or their combinations.

To further improve the stability, cans having a rolled-in rim and preferably a part or full rollover rim are used.

The formulation is actuated by a metering valve capable of delivering a volume of between 25 µl and 100 µl.

The choice of the metering valve and type of gasket will be made according the knowledge of persons skilled in the art. The gasket may comprise any suitable elastomeric material such as low density polyethylene, EPDM, chloroprene and TPE.

Suitable valves are commercially available from manufacturers well known in the aerosol industry, for example from Valois, France, Bespak p1c, UK and 3M, Neotechnic Ltd, UK.

The hydrofluorocarbon propellant is preferably selected from HFA 134a, HFA 227 and mixtures thereof.

The co-solvent can consist of one or more compounds and in this case their ratio in the formulation is a critical factor for an efficient aerosolization.

The preferred co-solvents are usually alcohols such as ethanol, propanol, propylene glycol, polyethylene glycol and glycerol in an amount up to 25% w/w, preferably up to 15% w/w, more preferably up to 10% w/w, even more preferably up to 5% w/w. The most preferred co-solvent is ethanol.

To reach the desired pharmaceutically useful concentration of the active ingredient in the formulation and the range of MMAD necessary for efficient delivery to the lungs and for the absorption into the blood stream, a careful selection of the co-solvent and of the ratio of propellant to the co-solvent is required.

Advantageously the size of the droplets is between about 0.5 µm and 2.5 µm corresponding to a MMAD of about 1-2 µm. The formulations of the inventions can be used for replacement therapy for oestrogen deficiency symptoms in postmenopausal women as well as for the treatment of menstrual migraine headaches.

The following examples further illustrate the invention.

By suitably applying the choice of the co-solvents and their ratio, a person skilled in the art would easily apply the teaching of the present invention to other synthetic analogues of 17β-oestradiol.

### EXAMPLES

### Experimental studies

### Preparation of HFA Solution pMDIs

The assembly of the pMDI cans was carried out using hand operated crimping and filling equipment. Formulations were prepared by accurately weighing the required quantity of drug into the can or vial. The appropriate volume of ethanol and the other co-solvent, if required, in the formulation was then added. The valve was crimped onto the vial/can and the assembled vial/can was ultra-sonicated for approximately 10 minutes. The HFA propellant was filled through the valve and the pMDI was ultra-sonicated for a further 10 minutes. In the case of formulations that only contained drug and propellant, the pMDI was ultra-sonicated once, after the propellant had been added. Final compositions were calculated as percentage w/v for the active ingredient and as percentage w/w for the co-solvents.

### Solubility Studies

All solubility investigations were conducted in plastic coated glass pMDI vials fitted with continuous spray valves. Once produced, the oestrogen-HFA solution pMDIs were stored in refrigerator at 4°C (± 0.1 °C). The pMDI vials were removed periodically and the vials assessed visually with the aid of a polarized light unit for the presence of crystals.

### Cascade Impaction Studies

All impaction studies were conducted with formulations contained in cut edge anodised aluminium cans fitted with 50µl or 100µl valves. The studies were carried out using an Andersen Cascade Impactor (ACI) fitted with a USP XXII metal throat entry port.

The ACI was operated at a flow rate of 28.3 ± 2 1 min ⁻¹. All solution HFA formulations were discharged into the ACI through a 0.25mm actuator. Deposition of the drug on each ACI plate was determined by high pressure liquid chromatography (HPLC).

MMAD values and corresponding geometric standard deviations (GSD) were calculated from plots of the cumulative percentage undersize of drug collected on each ACI plate (probit scale), against the upper cut off diameter for each respective ACI plate (log 10 scale).

The following parameters were determined: the metered dose, which is the sum of the dose delivered through the Andersen apparatus plus the active ingredient residue deposited on the device actuator; the cumulative amount of active particles deposited on the various ACI stages; the amount on the actuator; the amount in the adaptor and in the throat (adp/throat); the fine particle dose or respirable dose (FPD) which is the amount of particles deposited on stages 3 to filter of the ACI and corresponds to the amount of particles of size less than 4.7 µm; the fine particle fraction or respirable fraction which is the ratio between the respirable dose and the dose delivered ex-actuator.

### EXAMPLE 1

### SOLUBILITY STUDIES OF 17β-OESTRADIOL

The solubility of 17β-oestradiol was investigated by producing pMDI formulations containing 0.05-0.1% w/v of 17β-oestradiol and various percentages of ethanol in either HFA134 or HFA227. 17β-Oestradiol was found to be insoluble at all concentrations and percentages of ethanol investigated. The addition of glycerol and water were investigated with the aim of improving the solubility of 17β-oestradiol in the HFA134a.

Only by addition of small amounts of water (0.5% w/w) , was it possible to prepare a solution formulation containing 0.05% w/v of the active ingredient.

However, it is likely that the presence of water could lead to an unacceptable levels of drug degradation and/or could badly affect the homogeneity of the solution giving rise to physical stability problems. Moreover said concentration of 17β-oestradiol is unlikely to give the serum oestradiol levels necessary to produce a therapeutic effect.

### EXAMPLE 2

### SOLUBILITY STUDIES OF β-OESTRADIOL-17-VALERATE AND AEROSOL DELIVERY CHARACTERISTICS OF ITS CORRESPONDING PMDI FORMULATIONS

### Solubility studies

The solubility of β-oestradiol-17-valerate was investigated by producing pMDI formulations at various percentages of ethanol in HFA134a.

The results showed that a formulation containing 0.2% w/v β-oestradiol 17-valerate is soluble in about 15%w/w ethanol with HFA134a.

With 0.5% w/w propylene glycol added, 0.2% w/v β-oestradiol 17-valerate is soluble in 6% w/w ethanol with HFA134a. Glycerol has also been investigated as a further co-solvent in β-oestradiol 17-valerate formulations containing various percentages of ethanol and HFA134a. The results of these investigations are shown in table 1. With the aid of 0.1% w/w glycerol, it is possible to solubilise 0.1% w/v β-oestradiol 17-valerate in HFA 134a containing only 5% w/w ethanol, and 0.2% w/v in HFA 134a with 8% w/w ethanol.

**Table 1:**

| The solubility of β-oestradiol 17-valerate in HFA134a formulations with various percentages of ethanol and 0.1% w/w glycerol. | | |
|---|---|---|
| **β-Oestradiol 17-valerate** **(%, w/v)** | **Ethanol** **(%, w/w)** | **Solubility** |
| 0.05 | 5 | Soluble |
| 0.1 | 5 | Soluble |
| 0.2 | 7 | Soluble |
| 0.4 | 15 | Soluble |

The results show that β-oestradiol 17-valerate has a better solubility in HFA134a.

### Aerosol delivery characteristics studies

Three β-oestradiol 17-valerate HFA134a solution formulations containing ethanol and 0.5% w/w propylene glycol or 0.1% w/w glycerol were produced. The composition of the formulations are summarised in Table 2.

**Table 2:**

| Summary of β-oestradiol 17-valerate HFA134a solution formulations used in cascade impaction studies. | | | |
|---|---|---|---|
| | **β-Oestradiol 17-valerate** **(%, w/v)** | **Ethanol** **(%,w/w)** | **Other co-solvent** **(%, w/w)** |
| Formulation 1 | 0.2 | 8 | 0.1 glycerol |
| Formulation 2 | 0.1 | 10 | 0.5 propylene glycol |
| Formulation 3 | 0.1 | 5 | 0.1 glycerol |

Two ACI deposition determinations were performed with each formulation. Twenty shots were discharged into the ACI.

Table 3 summarizes the delivery characteristics of the oestradiol valerate formulations. These formulations have an MMAD of 1.25-1.4µm and therefore, according to the literature, should result in deep lung deposition and systemic absorption through the lungs (Newman *et al.,* In: Dalby, R.S., Byron, P. R. and Farr, S. J. Eds. *Respiratory Drug Delivery VI,* 1998, pp. 9-15, Interpharm press).

**Table 3:**

| Summary of the delivery characteristic of β-oestradiol 17-valerate formulations. | | | |
|---|---|---|---|
| | **Formulation 1** **(200 µg/dose)** | **Formulation 2** **(50 µg/dose)** | **Formulation 3** **(50 µg/dose)** |
| Metered dose, µg | 182.60 | 48.31 | 47.60 |
| Delivered dose, µg | 167.20 | 44.25 | 43.25 |
| Adp/throat, µg | 34.72 | 13.39 | 6.61 |
| FPD, µg | 128.94 | 29.36 | 35.80 |
| FPF, (%) | 70.6 | 60.8 | 75.2 |
| MMAD, µm | 1.40 | 1.25 | 1.40 |
| GSD | 1.85 | 2.60 | 1.70 |

### EXAMPLE 3

### SOLUBILITY STUDIES OF OTHER 17β-OESTRADIOL ESTERS AND AEROSOL DELIVERY CHARACTERISTICS OF THEIR CORRESPONDING pMDI FORMULATIONS

### Solubility studies

The solubility of other esters of 17β-oestradiol was investigated and the results are summarised in table 4. Only β-oestradiol 17-enanthate, which has the longest aliphatic side chain, was found to be soluble at 0.1 % w/v, in 5% w/w ethanol, 0.1% w/w glycerol and HFA134a.

**Table 4:**

| Solubility of various 17 β-oestradiol esters in ethanol and HFA134a in presence or not of 0.1% w/w of glycerol. | | | | |
|---|---|---|---|---|
| **Oestradiol ester** | **Conc.** **(%, w/v)** | **Ethanol** **(%, w/w)** | **Glycerol** **(%, w/w)** | **Solubility** |
| 3-Benzoate | 0.1 | 10 | - | Insoluble |
| 3-Benzoate | 0.1 | 13 | - | Insoluble |
| 17-Cypionate | 0.1 | 5 | 0.1 | Insoluble |
| 17-Propionate | 0.1 | 5 | - | Insoluble |
| 17-Enanthate | 0.1 | 5 | 0.1 | Soluble |
| 17-Enanthate | 0.2 | 5 | 0.1 | Soluble |

### Aerosol delivery characteristics studies

Two ACI deposition determinations were performed on a 0.1% w/v β-oestradiol 17-enanthate HFA134a solution formulation, corresponding to a 50 µg/dose, and containing 5% w/w ethanol and 0.1% w/w glycerol. Ten shots were discharged into the ACI. The distribution achieved was similar to that seen for the 0.1% w/v β-oestradiol 17-valerate formulation also containing 5%w/w ethanol and 0.1% glycerol. The fine particle dose (FPD) was 38.8 µg; the fine particle fraction (FPF) was 78.5%, with an MMAD of 1.5µm and GSD of 1.7. These values also closely match that of the β-oestradiol 17-valerate formulation. The results suggest that 17β-oestradiol ester/HFA134a solution formulation containing 5% w/w ethanol and 0.1% w/w glycerol will produce the same deposition characteristics whichever acylic residue is used .

### EXAMPLE 4

### SOLUBILITY STUDIES OF 17β-OESTRADIOL-3,17-DIPROPIONATE AND AEROSOL DELIVERY CHARACTERISTICS OF THE CORRESPONDING pMDI FORMULATIONS

### Solubility studies

0.4 % w/v of β-oestradiol-3,17-dipropionate can be dissolved in HFA134a without the use of ethanol as a co-solvent. With the addition of 5% w/w ethanol to the formulation the amount of β-oestradiol 3,17-dipropionate solubilised is increased to 1% w/v. Higher percentages of ethanol in the formulation result in a non-homogeneous system due to crystal formation. This non-homogeneity is thought to be due to an increase in the amount of residual water in the formulation as the amount of ethanol increases.

The addition of water to β-oestradiol 3,17-dipropionate formulations was also investigated. The addition of 0.3% w/w water to HFA134a formulations containing 0.05% w/v β-oestradiol dipropionate and either 8, 10 or 15% w/w ethanol resulted in a non-homogeneous solution formulation, due to water increasing the polarity of the liquid phase.

### Aerosol delivery characteristics studies

Two ACI deposition determinations were performed on 0.4% w/v β-oestradiol 3,17-dipropionate formulations correspon ding to 200 µg/dose and containing either 0, 2 or 5% w/w ethanol and HFA134a. Ten doses were discharged into the ACI. An HFA134a solution formulation containing 1% w/v β-oestradiol 3,17-dipropionate (500 µg/dose) and 5% w/w ethanol was also evaluated. The ACI deposition profile was similar in shape to that achieved with the 200µg/dose β-oestradiol 3,17-dipropionate / 5% w/w ethanol / HFA134a solution formulation. The delivery characteristics of all four formulations are summarised in Table 5.

The results show that all the formulations are very efficient, achieving over 75% fine particle fraction FPF_{<47 µm}. The 0.4% w/v β-oestradiol 3,17-dipropionate formulation with no ethanol resulted in a very small MMAD as shown by a high filter deposition. The 0.4% w/v β-oestradiol 3,17-dipropionate formulations with 2 and 5% w/w ethanol produced an identical MMAD, but the GSD of the 5% w/w ethanol formulation is smaller. This suggests that atomization of the 0% w/w ethanol formulation is highly efficient as it produces a larger amount of very small particles (<0.43 µm). The atomization of the 2% w/w ethanol formulation is also very efficient, as it produces as well large amounts of very small particles (<0.43 µm). However, for systemic delivery, the amount of such particles needs to be minimal, as many would be exhaled due to a short residency time in the lung. Thus the formulation containing 5% w/w ethanol produces the deposition characteristics more likely to achieve systemic delivery.

**Table 5:**

| Summary of delivery characteristics of 200 µg/50 µ1(0.4% w/v) and 500 µg/50 µl (1%) w/v β-oestradiol 3,17-dipropionate HFA 134a formulations. | | | | |
|---|---|---|---|---|
| | **200 µg/dose** **0% w/w ethanol** | **200µg/dose** **2% w/w ethanol** | **200µg/dose** **5% w/w ethanol** | **500µg/dose** **5% w/w ethanol** |
| Metered dose, µg | 178.88 | 172.95 | 170.80 | 456.85 |
| Delivered dose,µg | 153.85 | 159.65 | 154.70 | 408.25 |
| Adp/Throat, µg | 9.67 | 25.07 | 29.99 | 66.03 |
| FPD,µg | 139.45 | 124.20 | 118.62 | 321.70 |
| FPF, (%) | 78.0 | 71.6 | 69.2 | 70.4 |
| Dose ≤0.43µm,µg (%) | 48.35 (27.0) | 18.10 (10.5) | 5.36 (3.3) | 23.43 (5.1) |
| MMAD, µm | 0.90 | 1.50 | 1.50 | 1.90 |
| GSD | 2.60 | 2.10 | 1.80 | 1.85 |

## Claims

1. An aereosol solution pharmaceutical formulation comprising an ester of 3,17-dihydroxy oestratriene derivatives as active ingredient in a concentration of at least 0.01% w/v in a mixture comprising a hydrofluoroalkane propellant and one or more co-solvents.

2. A pharmaceutical formulation according to claim 1 **characterised in that** the concentration of the active ingredient is at least 0.05% w/w.

3. A pharmaceutical formulation according to claims 1-2 wherein the active ingredient is an ester of 3,17-dihydroxy oestratriene derivatives of formula I 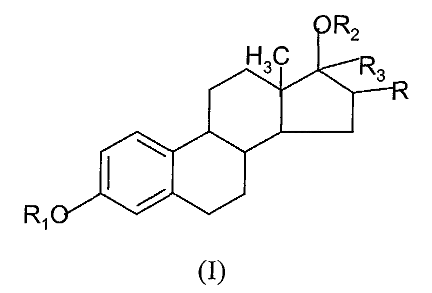 where
R is H or OH ;
R₁ is H, C₁-C₄ alkyl, C₁-C₁₀ alkanoyl or benzoyl;
R₂ is C₁ -C₁₀ alkanoyl or (C₅-C₆)cycloalkyl(C₁ -C₅) alkanoyl;
R₃ is H or ethynyl.

4. A pharmaceutical formulation according to claims 1-3, containing as the active ingredient a compound of formula I wherein R₁ and R₂ are C₂-C₇ alkanoyl groups.

5. A pharmaceutical formulation according to claims 1-3, containing as the active ingredient a compound of formula I wherein R₁ and R₂ are C₃ alkanoyl groups or wherein R₁ is H and R₂ has five or more carbon atoms.

6. A pharmaceutical formulation according to claim 5, wherein the active ingredient is selected from β-oestradiol-3,17,-dipropionate or β-oestradiol-17-valerate.

7. A pharmaceutical formulation according to any preceding claim, wherein the co-solvent is selected from ethanol, glycerol, propylene glycol, polyethylene glycol and their combinations.

8. A pharmaceutical formulation according to any preceding claim, wherein the propellant includes one or more HFAs selected from HFA 134a and HFA 227.

9. A pharmaceutical formulation according to any preceding claim, wherein the co-solvent is ethanol in an amount up to 10% w/w.

10. A method ot the preparation of the formulations of claims 1-8, the method comprising the steps of:
(a) weighing the required quantity of active ingredient into the can or vial;
(b) adding the appropriate volume of ethanol and a further cosolvent, if required;
(c) crimping with valves and gassing;
(d) adding a propellant containing a hydrofluoroalkane (HFA).

11. Use of an ester of 3,17-dyhdroxy oestratriene derivatives for preparing pharmaceutical compositions of claims 1-9 for the treatment of postmenopausal symptoms and menstrual migraine headaches.

12. Use of an ester according to claim 11 wherein the 3,17-dihydroxy oestratriene derivative is 17β-oestradiol.

13. A metered dose aerosol inhaler comprising the solution formulations of claims 1-9.
